(19) Europäisches Patentamt
European Patent Office
Office européen des brevets

(11) Publication number: 0 015 756 B1

(12) **EUROPEAN PATENT SPECIFICATION**

(45) Date of publication of patent specification: 04.05.83

(21) Application number: 80300693.1

(22) Date of filing: 06.03.80

(51) Int. Cl.³: C 07 D 249/08, A 01 N 43/64 //(C07D303/28, C07C31/34, 29/40)

(54) **Triazole compounds, a process for preparing them, their use as plant fungicides and fungicidal compositions containing them.**

(30) Priority: 07.03.79 GB 7908003
21.09.79 GB 7932819

(43) Date of publication of application: 17.09.80 Bulletin 80/19

(45) Publication of the grant of the patent: 04.05.83 Bulletin 83/18

(84) Designated Contracting States: BE CH DE FR GB IT NL

(56) References cited:
EP - A - 0 011 769
GB - A - 1 529 818
US - A - 3 394 143

(73) Proprietor: IMPERIAL CHEMICAL INDUSTRIES PLC
Imperial Chemical House Millbank
London SW1P 3JF (GB)

(72) Inventor: Parry, Keith Peter
10, Calder Court
Maidenhead Berkshire (GB)
Inventor: Worthington, Paul Anthony
22, Boulters Gardens
Maidenhead Berkshire (GB)
Inventor: Rathmell, William George
'Culvers' 10, Gypsy Lane
Wokingham Berkshire (GB)

(74) Representative: H G H Alner
Imperial Chemical Industries PLC Legal Department: Patents Thames House North Millbank
London SW1P 4QG (GB)

**The file contains technical information submitted after the application was filed and not included in this specification**

EP 0 015 756 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Triazole compounds, a process for preparing them, their use as plant fungicides and fungicidal compositions containing them

This invention relates to triazole compounds useful as fungicides, to a process for preparing them, to fungicidal compositions containing them, and to a method of combating fungal infections in plants using them.

In U.K. Patent No. 1529818 there are described and claimed compounds of formula

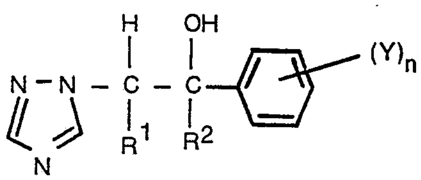

wherein $R^1$ and $R^2$ are hydrogen or optionally substituted hydrocarbyl; Y is hydrogen, halogen, nitro, alkyl, alkoxy, or optionally substituted amino; and n is an integer of 0 to 5; and salts thereof.

In the foregoing patent no compounds are disclosed, however wherein $R^1$ is hydrogen, and, with the exception of one single compound, all the compounds specified had $R^2$ as hydrogen.

Most surprisingly it has now been discovered that by selecting compounds in which $R^1$ is hydrogen, and in which $R^2$ has certain values other than hydrogen, compounds are obtained which have considerably higher fungicidal activity. Indeed the increased activity is of a different order in magnitude.

The triazole compounds have the general formula (I):—

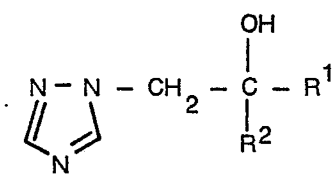

wherein $R^1$ is an alkyl group containing from 1 to 6 carbon atoms, cycloalkyl or phenyl optionally substituted with halogen, an alkyl group containing from 1 to 5 carbon atoms, an alkoxy group containing from 1 to 4 carbon atoms, trifluoromethyl, nitro or phenoxy; $R^2$ is phenyl or benzyl optionally substituted with halogen, an alkyl group containing from 1 to 5 carbon atoms, an alkoxy group containing from 1 to 4 carbon atoms, trifluoromethyl, nitro or phenoxy; or $R^2$ is phenylphenyl but only when $R^1$ is alkyl or cycloalkyl; and acid addition salts and metal complexes thereof.

The compounds of the invention can contain chiral centres. Such compounds are generally obtained in the form of racemic mixtures. However, these and other mixtures can be separated into the individual isomers by methods known in the art.

The alkyl groups can be a straight or branched chain group having 1 to 6, e.g. 1 to 4, carbon atoms; examples are methyl, ethyl, propyl (*n*- or *iso*-propyl) and butyl (*n*-, *sec*-, *iso*- or *t*-butyl).

Examples of suitable substitutents for the phenyl and for the phenyl moiety of the benzyl are halogen (e.g. fluorine, chlorine or bromine), $C_{1-5}$ alkyl [e.g. methyl, ethyl propyl (*n*-or *iso*-propyl) and butyl (*n*-, *sec*-, *iso*- or *t*-butyl)], $C_{1-4}$ alkoxy (e.g. methoxy and ethoxy), trifluoromethyl, nitro, phenyl and phenoxy. The alkyl moiety of the benzyl can be substituted with for example one alkyl (e.g. methyl or ethyl). Suitably the phenyl and benzyl are unsubstituted or substituted with 1, 2 or 3 ring substituents as defined above. Preferably the benzyl and phenyl have a single ring substituent in the *o*-, m-, or *p*-position. Examples of these groups are phenyl, benzyl, $\alpha$-methylbenzyl, o-, m- or p-chlorophenyl, 2,4- or 2,6-dichlorophenyl, o-, m- or p-fluorophenyl, 2,6-difluoro-phenyl, o-, m- or p-bromophenyl, 2-chloro-4-fluorophenyl, 2-chloro-6-fluorophenyl, o-, m- or p-methoxyphenyl, 2,4-dimethoxyphenyl, o-, m- or p-ethoxyphenyl, o-, m- or p-nitrophenyl, o-, m- or p-methylphenyl, o-, m- or p-*t*-butylphenyl, o-, m- or p-trifluoromethylphenyl, o-, m- or p-phenoxyphenyl, and o-, m- or p-phenylphenyl (o-, m- or p-biphenylyl), and the corresponding ring substituted benzyl and $\alpha$-methylbenzyl groups.

The invention, in a further aspect, provides compounds of formula I above as defined, and wherein $R^1$ is $C_{1-4}$ alkyl and $R^2$ is optionally substituted benzyl.

In a still further aspect the invention provides compounds of formula I above as defined, and, wherein $R^1$ is optionally substituted phenyl and $R^2$ is optionally substituted benzyl.

In another aspect the invention provides compounds of formula I above as defined, and wherein each of $R^1$ and $R^2$, which may be the same or different, is optionally substituted phenyl.

In yet another aspect the invention provides compounds of formula I above as defined, and wherein $R^1$ is $C_{1-4}$ alkyl and $R^2$ is optionally substituted phenyl.

The invention further provides compounds of formula I above, as defined and wherein $R^1$ is *t*-butyl and $R^2$ is optionally substituted phenyl or optionally substituted benzyl.

The salts can be salts with inorganic or organic acids e.g. hydrochloric, nitric, sulphuric, acetic, p-toluenesulphonic or oxalic acid. Suitably the metal complex is one including, as the metal, copper, zinc, manganese or iron. It preferably has the general formula:

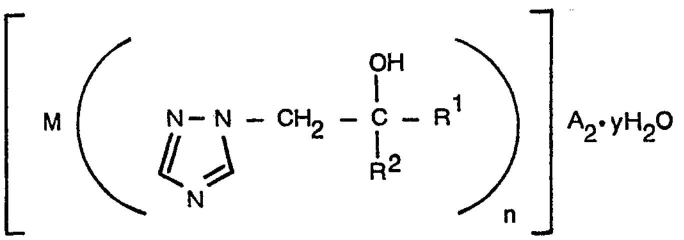

wherein Y, $R^1$ and $R^2$ are as defined above, M is a metal, A is an anion (e.g. a chloride, bromide, iodide, nitrate, sulphate or phosphate anion), n is 2 or 4 and Y is 0 or an integer of 1 to 12.

Examples of the compounds of the invention are shown in Table I.

TABLE I

| Compound No. | $R^1$ | $R^2$ | Melting Point (°C) |
|---|---|---|---|
| 1 | $C_6H_5-$ | $C_6H_5CH_2-$ | 124–125 |
| 2 | $C_6H_5-$ | $p\text{-}Cl-C_6H_4CH_2-$ | 144–145 |
| 3 | $C_6H_5-$ | $p\text{-}F-C_6H_4CH_2-$ | 116–118 |
| 4 | $p\text{-}Cl-C_6H_4-$ | $p\text{-}Cl-C_6H_4CH_2-$ | 80–83 |
| 5 | $p\text{-}Cl-C_6H_4-$ | $C_6H_5CH_2-$ | 109–111 |
| 6 | $p\text{-}F-C_6H_4-$ | $C_6H_5CH_2-$ | 141–142 |
| 7* | $C_6H_5-$ | $2,4\text{-}diCl-C_6H_3CH_2-$ | 104–106 |
| 8† | $p\text{-}F-C_6H_4-$ | $p\text{-}F-C_6H_4CH_2-$ | 154–156 |
| 9 | $p\text{-}F-C_6H_4-$ | $p\text{-}Cl-C_6H_4CH_2-$ | 168–170 |
| 10 | t-Bu | $C_6H_5CH_2-$ | 110–110 |
| 11 | t-Bu | $p\text{-}Cl-C_6H_4CH_2$ | 86–87 |
| 12 | t-Bu | $p\text{-}F-C_6H_4CH_2-$ | 146–148 |
| 13 | $C_6H_5-$ | $o\text{-}F-C_6H_4CH_2-$ | 133–134 |
| 14 | $p\text{-}Cl-C_6H_4-$ | $o\text{-}F-C_6H_4CH_2-$ | 95–96 |
| 15 | $C_6H_5-$ | $o\text{-}Cl-C_6H_4CH_2$ | 69–71 |
| 16 | $p\text{-}MeO-C_6H_4-$ | $C_6H_5CH_2$ | 100–103 |
| 17 | $C_6H_5-$ | $C_6H_5-$ | 128–129 |
| 18‡ | $p\text{-}F-C_6H_4-$ | $p\text{-}F-C_6H_4CH_2-$ | 161–163 |
| 19 | $C_6H_5-$ | $2,4\text{-}diCl-C_6H_3CH_2-$ | 104–106 |
| 20 | t-Bu | $o\text{-}Cl-C_6H_4CH_2-$ | 74–75 |
| 21 | t-Bu | $o\text{-}F-C_6H_4CH_2-$ | 96–98 |
| 22 | t-Bu | $m\text{-}Cl-C_6H_4CH_2-$ | 88–89 |
| 23 | t-Bu | $m\text{-}CF_3-C_6H_4CH_2-$ | 106–107 |
| 24 | $C_6H_5-$ | $p\text{-}t\text{-}Bu-C_6H_4CH_2-$ | 80–83 |
| 25 | $p\text{-}Cl-C_6H_4-$ | $C_6H_5-$ | 83–85 |
| 26 | $p\text{-}Cl-C_6H_4-$ | $p\text{-}Cl-C_6H_4-$ | 147–148 |
| 27 | $p\text{-}Cl-C_6H_4-$ | $p\text{-}F-C_6H_4-$ | 154–155 |
| 28 | $2,4\text{-}diCl-C_6H_3-$ | $C_6H_5-$ | 191–194 |
| 29 | $p\text{-}F-C_6H_4-$ | $p\text{-}F-C_6H_4-$ | 170–171 |